# EUROPÄISCHE PATENTSCHRIFT

(11) **EP 2 758 182 B1**
(45) Veröffentlichungstag und Bekanntmachung des Hinweises auf die Patenterteilung: **17.04.2019**
(21) Anmeldenummer: 12759476.0
(22) Anmeldetag: 19.09.2012
(51) Int. Cl.: B05C 17/00, B65D 47/42, B43M 11/06, A61F 13/40, A45D 34/04

(54) **APPLIKATIONSSTIFT**
APPLICATOR
CRAYON D'APPLICATION

(30) Priorität: 23.09.2011 EP 11182576
(43) Veröffentlichungstag der Anmeldung: 30.07.2014
(73) Patentinhaber: Sika Technology AG, 6340 Baar (CH)
(72) Erfinder: MAIWALD, Dana, CH-8006 Zürich (CH); BURKARD, Dominique, CH-5014 Gretzenbach (CH)
(74) Vertreter: Sika Patent Attorneys
(86) Internationale Anmeldenummer: PCT/EP2012/068449
(87) Internationale Veröffentlichungsnummer: WO 2013/041573

(56) Entgegenhaltungen:
- WO-A1-2010/003518
- FR-A1- 2 769 506
- US-A- 2 832 087
- US-A- 2 967 319
- US-A- 3 152 352
- US-A- 4 957 385
- US-A1- 2005 054 967
- US-A1- 2005 111 900

## Beschreibung

### Technisches Gebiet

Die Erfindung betrifft einen Applikationsstift zum, insbesondere punktuellen oder flächigen, Austrag einer Flüssigkeit, mit einem hohlen Stiftkörper aus Kunststoff mit einem geschlossenen und einem offenen Ende und einem am offenen Ende des Stiftkörpers sitzenden Applikatorkopf, der ein poröses Material, insbesondere aus Schaumstoff, Filz, Faserkörper oder porösem Kunststoff aufweist.

### Stand der Technik

Vorbehandlungsmittel zur Erzeugung von Klebeverbindungen und Korrosionsschutz werden üblicherweise in Verpackungen angeboten, die aus einer Aluminiumflasche, einem Polyethylen(PE)-Napf und einem Schraubverschluss der Flasche aus Polypropylen (PP) bestehen. Im Bereich der Einmal-Anwendungen werden auch Aluminiumtuben oder Primer-Stifte und Aktivator-Pads mit jeweils sehr geringem Inhalt angeboten.

Auch Applikationsstifte der oben beschriebenen Art sind seit längerem im Handel und im praktischen Einsatz. Der Applikatorkopf ist bei solchen Stiften üblicherweise mit dem Stiftkörper verschweißt, was einen thermischen Prozessschritt bei der Herstellung erfordert, der gewisse Nachteile hinsichtlich der Prozessführung mit sich bringt. Aus der US 2005/0191113 A1 ist eine konstruktive Ausgestaltung des Applikationsstiftes bekannt, bei der das offene Ende eine durch Erwärmung erzeugte Einschnürung aufweist, mittels derer der (in gleicher Weise eingeschnürte) Applikatorkopf auf dem Stiftkörper gehalten und zugleich eine gewisse Kontrollfunktion bezüglich des Flüssigkeitsaustrags aus dem Applikationsstift ausgeübt wird.

Aus der US/2005/0111900 A1 ist eine Ampulle und deren Verwendung bekannt. Dabei wird ein erster zerbrechlicher Container mit einer Flüssigkeit in einem zweiten Container angeordnet, wobei der zweite Container einen Applikator zur Applikation der Flüssigkeit aufweist.

Der Erfindung liegt daher die Aufgabe zugrunde, einen verbesserten Applikationsstift der gattungsgemäßen Art bereitzustellen, der insbesondere kostengünstig herzustellen und leicht, flexibel und sicher zu benutzen ist.

### Offenbarung der Erfindung

Diese Aufgabe wird durch einen Applikationsstift mit den Merkmalen des Anspruchs 1 gelöst. Aus der US 2005/0111900 A1 ist ein Applikationsstift nach der Präambel des Anspruchs 1 bekannt. Zweckmäßige Fortbildungen des Erfindungsgedankens sind Gegenstand der abhängigen Ansprüche.

Die Erfindung schließt die Überlegung ein, von einer den Applikatorkopf erheblich deformierenden und den Flüssigkeitsstrom aus dem Stiftkörper einschränkenden Einschnürung am offenen Ende des Stiftkörpers zur Fixierung des Applikatorkopfes abzugehen. Sie schließt weiter den Gedanken ein, stattdessen die benötigte Haltekraft auf die Außenwandung des Applikatorkopfes ohne wesentliche Deformation desselben aufzubringen, und zwar speziell über den größeren Teil des in das offene Ende des Stiftkörpers ragenden Endabschnitts. Demgemäß sind am Stiftkörper Fixierungsmittel vorgesehen, die in oberflächlichem Form- und/oder Reibschluss mit der Außenoberfläche des Applikatorkopfes stehen.

In zweckmäßigen Ausführungen der Erfindung weist der Applikatorkopf oder jedenfalls sein Endabschnitt im eingesetzten Zustand eine prismatische oder zylindrische Grundform auf. Es kann sich hierbei insbesondere um die Form eines an den Kanten abgerundeten Drei-, Vier- oder Sechskantprismas handeln, und dieses kann am freien Ende des Applikatorkopfes abgeschrägt oder (für einen eher linearen Flüssigkeitsaustrag) angespitzt sein. Es versteht sich, dass Teile oder Abschnitte des Stiftkörpers als Fixierungsmittel zur Herstellung des erwähnten Formschlusses in diese Grundform eindringen können, jedoch soll dieses Eindringen die Grundform nicht wesentlich verändern.

Nach der Erfindung ist vorgesehen, dass mindestens der Endabschnitt des Applikatorkopfes und mindestens das offene Ende des Stiftkörpers zylindrisch geformt sind und die Fixierungsmittel ein Innengewinde am Stiftkörper aufweisen, welches in den Umfang des Endabschnitts des Applikatorkopfes einschneidet. Speziell können die Gewindegänge im Querschnitt im wesentlichen dreieckig oder trapezförmig sein. Bei dieser Ausführung wird der Applikatorkopf nicht axial in den Stiftkörper geschoben, sondern in diesen mit einer Drehbewegung eingesetzt, die ein Einschneiden oder Einpressen des Gewindes in die Oberfläche des Applikatorkopfes bewirkt. Speziell begrenzt hierbei ein anschlag-Rand an der Innenwandung des Stiftkörpers die Eindringtiefe des Applikatorkopfes.

In einer anderen nicht erfindungsgemäßen Variante sind an die Innenwandung des Stiftkörpers nahe dem offenen Ende inselartige Fortsätze angeformt, die in den Umfang des Endabschnitts des Applikatorkopfes eingreifen und insbesondere widerhakenartig wirken, derart, dass sie durch den Applikatorkopf beim Einschieben leicht überwunden werden können, seinem Herausziehen aber hohen Widerstand entgegensetzen. Je nach konkreter Formgestaltung der Fortsätze kann hier das Einführen des Applikatorkopfes in das offene Stiftkörper-Ende axial oder wiederum drehend erfolgen.

In einer weiteren nicht erfindungsgemäßen Variante umfassen die Fixierungsmittel am offenen Ende des Stiftkörpers angeformte Krallen, die im Ausgangszustand zum Einführen des Endabschnitts des Applikatorkopfes aufgespreizt sind. Des Weiteren umfassen sie eine Verschlusshülse, die auf die Krallen in deren aufgespreiztem Zustand aufschiebbar und derart geformt ist, dass sie die Krallen beim Aufschieben in die Außenwandung des Endabschnitts des Applikatorkopfes punktuell eindrückt, ohne seinen Querschnitt und damit den Fließquerschnitt der im Stift enthaltenen Flüssigkeit wesentlich zu verringern. In einer ersten Ausgestaltung ist die Verschlusshülse über Sollbruchstellen am Stiftköper angeformt. In einer zweiten Ausgestaltung ist die Verschlusshülse als separates Teil ausgebildet und insbesondere zusammen mit dem Applikationsstift verpackt.

Ähnlich der vorgenannten Variante ist eine weitere, nicht erfindungsgemäße Variante bei der anstelle von Krallen Zweige mit nach innen gerichteten inselartigen Fortsätzen im Ausgangszustand aufgespreizt vorliegen und mittels einer Verschlusshülse an den Außenumfang des Endabschnitts des Applikatorkopfes angedrückt werden. Die dabei in das Material des Applikatorkopfes eindringenden inselartigen Fortsätze wirken ähnlich wie die vorstehend erwähnten Krallen.

In einer praktisch besonders bedeutsamen Ausführung ist der Stiftkörper zur Aufnahme einer harten Ampulle ausgebildet und seine Wandung hinreichend flexibel, um die im Stiftkörper aufgenommene Ampulle zu brechen und hierdurch einen Austritt der Flüssigkeit aus der Ampulle in das Innere des Stiftkörpers zu bewirken. Derartige Applikationsstifte sind insbesondere zum Austrag von Primern oder ähnlichen Vorbehandlungsmitteln für Korrosionsschutz und Verklebungen etwa auf dem Gebiet der Herstellung von Autoverglasungen von hoher Marktrelevanz.

In einer vorteilhaften Ausgestaltung dieser Ausführung ist die Außenoberfläche der Wandung des Stiftkörpers mit mindestens einer, insbesondere ringförmigen, Vertiefung und/oder Erhebung strukturiert. Spezieller weist die Außenoberfläche der Wandung des Stiftkörpers eine Mehrzahl von ringförmigen Vertiefungen auf, welche einen Einschnürungsbereich des Stiftkörpers markieren. In diesem Einschnürungsbereich soll günstigerweise die Wandung des Stiftkörpers mit den Fingern eingedrückt und/oder gebogen oder auch über eine Kante eines harten Gegenstandes (etwa eine Tischkante) gebogen werden, um die darin aufgenommene Ampulle zu zerbrechen. Alternativ hierzu oder in Kombination mit der vorgenannten Ausgestaltung können in die Außenoberfläche der Wandung des Stiftkörpers Symbole eingeformt sein, welche eine Benutzerführung zur Ausübung von lokalem Druck auf den Stiftkörper zum Brechen der Ampulle bewirken.

In einer weiteren Ausführung ist nahe dem offenen Ende des Stiftkörpers eine Mehrzahl von ringförmigen Vertiefungen oder ähnlichen Strukturelementen vorgesehen, welche einen Greifbereich des Applikationsstiftes bestimmen. Die in das Material des Stiftkörpers eingearbeiteten Hinweis-Elemente können das zusätzliche Aufkleben oder Aufdrucken entsprechender Markierungen oder Hinweise überflüssig machen und somit die Herstellungskosten senken.

### Beschreibung der Zeichnungen

Vorteile und Zweckmäßigkeiten der Erfindung werden aus der nachfolgenden Beschreibung bevorzugter Ausführungsbeispiele anhand der Figuren deutlich. Von diesen zeigen:
- Fig. 1: eine Prinzipskizze zur Erläuterung der Erfindung, in Art einer Längsschnittdarstellung eines Applikationsstiftes,
- Fig. 2: eine Detailansicht (Längsschnittdarstellung) des offenen Endes des erfindungsgemäßen Applikationsstiftes,
- Fig. 3: eine Detailansicht (Längsschnittdarstellung) eines nicht erfindungsgemäßen Applikationsstiftes,
- Fig. 4A und 4B: eine perspektivische Teilansicht sowie eine Längsschnittdarstellung eines weiteren nicht erfindungsgemäßen Applikationsstiftes,

- Fig. 5A und 5B: zwei perspektivische Darstellungen eines weiteren nicht erfindungsgemäßen Applikationsstiftes,
- Fig. 6A und 6B: eine perspektivische Gesamtansicht bzw. Detailansicht zweier weiterer Ausführungen des erfindungsgemäßen Applikationsstiftes,
- Fig. 7: eine perspektivische Gesamtansicht bzw. Detailansicht einer weiteren Ausführung des erfindungsgemäßen Applikationsstiftes,
- Fig. 8: eine perspektivische Gesamtansicht bzw. Detailansicht einer weiteren Ausführung des erfindungsgemäßen Applikationsstiftes,
- Fig. 9: skizzenartige Darstellung verschieden geformter Applikatorköpfe, mit denen die Erfindung realisiert werden kann.

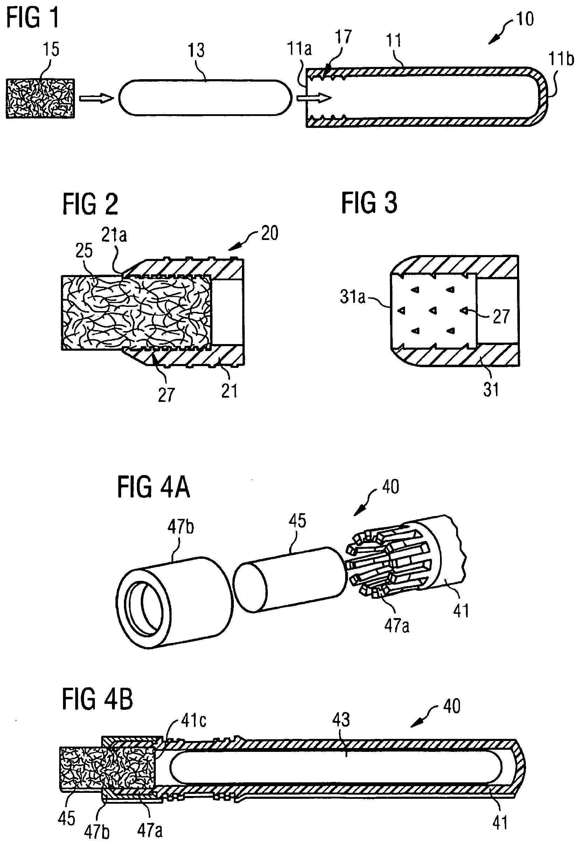

Fig. 1 zeigt skizzenartig in Art einer Längsschnittdarstellung einen Applikationsstift 10 zum Austrag eines Primers als Korrosionsschutz zur Vorbereitung von Fahrzeugscheiben-Verklebungen, der einen im wesentlichen steifen, aber eine gewisse Verformbarkeit aufweisenden Stiftkörper 11 aus thermoplastischem Kunststoff, eine Glasampulle 13, die den Primer enthält, zum Einsetzen in den Stiftkörper 11 und einen Applikatorkopf 15 zum punktuellen oder flächigen Applizieren des Primers umfasst, der aus einem porösem Material, insbesondere porösem Kunststoff, Schaumstoff, einem Filz oder Faserkörper besteht. Die dargestellten Teile können eine im Wesentlichen zylindrische oder auch prismatische Grundform haben. Der Stiftkörper 11 hat ein offenes Ende 11a und ein geschlossenes Ende 11b, und an seiner Innenwandung sind nahe dem offenen Ende form- bzw. reibschlüssig wirkende Fixierungsmittel 17 für den Applikatorkopf 15 vorgesehen. Eine Ausführungsform dieser Fixierungsmittel wird weiter unten beschrieben. In einer hier nicht gezeigten Abwandlung hat der Stiftkörper am geschlossenen Ende hinten einen kleinen Fortsatz, der die Einschubtiefe der Ampulle begrenzt.

Fig. 2 zeigt als Ausführungsform nur das offene Ende 21a des Stiftkörpers 21 des erfindungsgemäßen Applikationsstiftes 20 mit eingesetztem Applikatorkopf 25. Hier ist in die Innenwandung des Endbereiches des Stiftkörpers 21 als Fixierungsmittel für den Applikatorkopf 25 ein Innengewinde 27 eingearbeitet, und der Applikatorkopf 25 wird durch Eindrehen vom offenen Ende her mit dem Stiftkörper verbunden, wobei das Gewinde 27 in seine Außenwandung einschneidet. Am Ende des Gewindes 27 ist der Innendurchmesser des Stifrkörpers 21 etwas reduziert, wodurch (in gleicher Weise wie unten zu Fig. 4B beschrieben) ein Anschlag für den Applikatorkopf 25 gebildet ist.

Fig. 3 zeigt einen nicht erfindungsgemäßen Stiftkörper 31, nahe dessen offenem Ende als Fixierungsmittel zum hinreichend festen Einfügen eines (hier nicht gezeigten) Applikatorkopfes inselförmige Fortsätze 27 vorgesehen sind, die im Querschnitt wie in der Draufsicht annähernd dreieckförmige Gestalt haben und in einen eingeschobenen Applikatorkopf widerhakenartig eingreifen.

Fig. 4A und 4B zeigen in einer perspektivischen Explosionsdarstellung (Teilansicht) bzw. einer Längsschnittdarstellung einen nicht erfindungsgemäßen Applikationsstift 40. Ein annähernd zylindrischer Stiftkörper 41 enthält eine Ampulle 43, und an seinem vorderen Ende ist ein Filz-Applikatorkopf 45 mittels eines Krallen-Ringes 47a und einer Verschlusshülse 47b fixiert. In Fig. 4A ist ein Ausgangszustand des Stiftkörpers 41 gezeigt, wie er im Formvorgang erzeugt wird und bei dem die Krallen 47a leicht nach außen abgespreizt sind. Dies ermöglicht ein praktisch widerstandsfreies Einschieben des Applikatorkopfes 45 in das offene Ende des Stiftkörpers. Nach dem Einschieben, wobei die Einschubtiefe durch einen als Anschlag wirkenden Ringflansch 41c oder durch die Ampulle im Stiftkörper begrenzt wird, wird die separat gefertigte Verschlusshülse 47b ebenfalls vom offenen Ende her über den Krallenring 47a geschoben und drückt die Krallen nach innen, in das Material des Applikatorkopfes 45. Die Verschlusshülse 47b hält ihrerseits Reibschluss mit den Außenoberflächen der Krallen und hält dadurch am Stiftende, oder es ist ein Gewindegang oder Bajonettverschluss o. ä. vorgesehen.

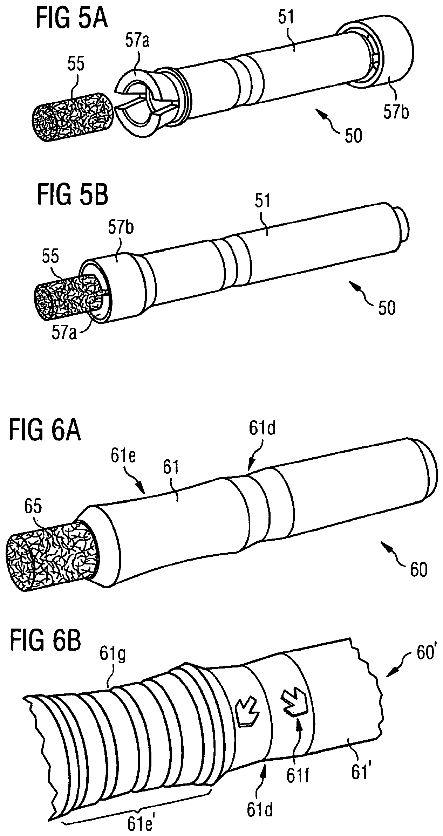

Fig. 5A und 5B zeigen als gegenüber der Ausführung nach Fig. 4A und 4B modifizierte nicht erfindungsgemäße Variante einen weiteren nicht erfindungsgemäßen Applikationsstift 50 in perspektivischer Darstellung im Ausgangszustand bzw. im Gebrauchszustand. Anstelle eines Krallenringes sind hier zwei krallenartig wirkende, im Ausgangszustand aufgespreizte Halbringe 57a an das offene Ende des Stiftkörpers 51 angeformt. An sein geschlossenes Ende ist eine Verschlusshülse 57b über (nicht gesondert bezeichnete) Kunststoffstege angeformt, die leicht zu brechen sind und nach Brechen die Verschlusshülse 57b freigeben. Nachdem der Applikatorkopf 55 in das offene Ende des Stiftkörpers eingeführt wurde, wird von dessen geschlossenem Ende her die vom Stiftkörper gelöste Verschlusshülse 57b in Richtung auf das offene Ende vorgeschoben, greift dabei in die Halbringe 57a ein und drückt diese an den Umfang des Applikatorkopfes 55 an bzw. mit den etwas nach innen vorstehenden Vorderkanten in dessen Material geringfügig ein. Hierdurch wird in dem in Fig. 5B zeigten Gebrauchszustand der Applikatorkopf durch Formschluss am Stiftkörper 51 gehalten.

Fig. 6A zeigt einen weiteren Applikationsstift 60, dessen Grundform wie bei den vorstehend beschriebenen Applikationsstiften (ebenso wie die Grundform des Applikatorkopfes 65) zylindrisch ist. Eine umlaufende Einkerbung 61d in der Außenwandung signalisiert dem Benutzer im Sinne einer Benutzerführung, dass dort manuell Druck anzuwenden oder ggfs. ein Werkzeug anzusetzen ist, um die im Stiftkörper enthaltene (nicht gezeigte) Ampulle zu zerbrechen und hierdurch den Austritt der darin enthaltenen Flüssigkeit in das innere des Stiftkörpers zu bewirken. Darüber hinaus ist der Stiftkörper in seiner vorderen Hälfte, an deren Ende der Applikatorkopf 65 angeordnet ist, mit einer großflächigen Einwölbung 61e versehen. Hier soll der Benutzer den Stift ergreifen, und die Einwölbung erleichtert das Ergreifen und Führen des Stiftes über eine mit der Flüssigkeit zu beschichtende Fläche.

Fig. 6B zeigt als modifizierte Ausführung in Detailansicht den mittleren Bereich eines weiteren Applikationsstiftes 60', bei dem beidseits der oben erwähnten Einkerbung 61d Pfeil-Symbole 61f in die Außenwandung des Stiftkörpers 61' eingeformt sind. Diese sollen dem Benutzer zusätzlich signalisieren, dass und in welche Richtung er hier manuellen Druck aufbringen soll. Der weiter oben erwähnte Einwölbungsbereich im vorderen Teil des Stiftkörpers ist hier zusätzlich mit ringförmigen Vertiefungen 61g versehen und zur Unterscheidung von dem unstrukturierten Einwölbungsbereich 61e des Applikationsstiftes 60 nach Fig. 6A mit der Bezugsziffer 61e' bezeichnet. Die zusätzlichen ringförmigen Ausnehmungen 61g erleichtern das Halten des Applikationsstiftes, wenn der Benutzer z.B. Handschuhe trägt oder der Stift feucht ist.

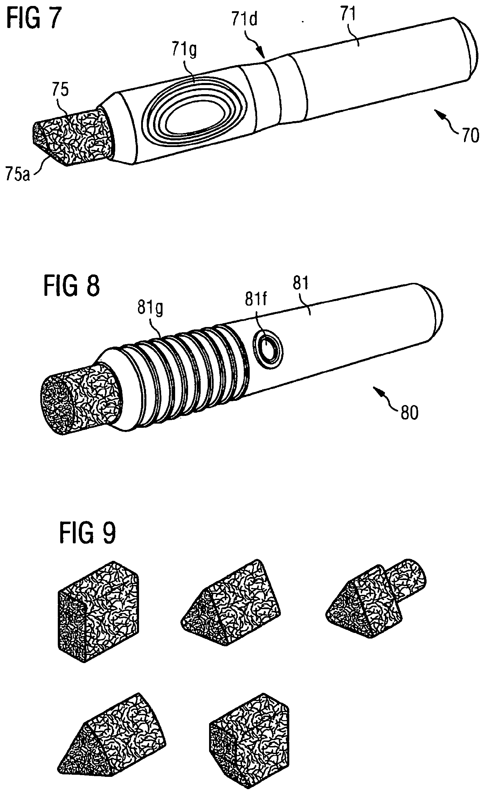

Fig. 7 zeigt einen weiteren Applikationsstift 70, dessen Gestaltung ähnlich der in Fig. 6A gezeigten und oben beschriebenen ist, dessen Griffbereich aber durch ellipsenförmige Erhebungen 71g strukturiert ist, die sich - anders als die ringförmigen Vertiefungen 61g der Ausführung nach Fig. 6B - nicht über den Umfang des Stiftes erstrecken, sondern jeweils seitlich längs der Wandung verlaufen. Darüber hinaus ist hier ein Applikatorkopf 75 eingesetzt, dessen Grundform zwar zylindrisch ist, der an seinem freien Ende jedoch eine Schräge 75a aufweist.

Fig. 8 zeigt einen weiteren Applikationsstift 80, bei dem die zylindrische Außenform des Stiftkörpers 81 über dessen gesamte Länge (mit Ausnahme der unmittelbaren Nachbarschaft des offenen Endes) beibehalten ist. Hier sind im vorderen Bereich erhabene Ringe 81g zum leichteren Ergreifen und Führen des Stiftes angeformt, und im mittleren Bereich gibt es (beidseits) ringförmige Markierungen 81f, die dem Nutzer einen geeigneten Druckpunkt zum Zerbrechen der im Stiftkörper aufgenommen Ampulle anzeigen sollen.

In Fig. 9 sind skizzenartig verschiedene Ausführungen von Applikatorköpfen dargestellt, die in einem erfindungsgemäßen Applikationsstift zum Einsatz kommen können. Die Darstellungen sind selbsterklärend, so dass hier auf eine verbale Beschreibung verzichtet werden kann. Es versteht sich, dass die Formgebung zumindest im Bereich des offenen Endes eines zugehörigen Stiftkörpers an die Außenform des Endes des jeweiligen Applikatorkopfes angepasst sein muss. Für den übrigen Teil des Stiftkörpers kann die Außenform jedoch abweichen, ebenso wie die Grundform des Endabschnitts des Applikatorkopfes eine andere sein kann als die Form im Bereich seines freien Endes, mit dem der Flüssigkeitsauftrag auf eine Oberfläche erfolgt.

Die Ausführung der Erfindung ist nicht auf diese Beispiele beschränkt, sondern ebenso in einer Vielzahl von Abwandlungen möglich, die im Rahmen der Ansprüche liegen.

## Patentansprüche

1. Applikationsstift (10 - 80) zum, insbesondere flächigen, Austrag einer Flüssigkeit, mit einem hohlen Stiftkörper (11 - 81) aus Kunststoff mit einem geschlossenen und einem offenen Ende und einem am offenen Ende des Stiftkörpers sitzenden Applikatorkopf (15; 25; 45; 55; 65; 75), der ein poröses Material, insbesondere einen Schaumstoff, Filz oder Faserkörper, aufweist, wobei der Applikatorkopf einen in das offene Ende des Stiftkörpers passenden Endabschnitt hat und am Stiftkörper mechanische Fixierungsmittel (17; 27; 47a; 47b; - 57a; 57b) zum formschlüssigen und/oder reibschlüssigen Eingriff mit dem Außenumfang des Endabschnitts des Applikatorkopfes vorgesehen und derart ausgebildet sind, dass sie den Form- und/oder Reibschluss ohne wesentliche Deformation des in das offene Ende des Stiftkörpers ragenden Endabschnitts des Applikatorkopfes bewirken, wobei mindestens der Endabschnitt des Applikatorkopfes (25) und mindestens das offene Ende des Stiftkörpers (21) zylindrisch geformt sind und die Fixierungsmittel ein Innengewinde (27) am Stiftkörper aufweisen, **dadurch gekennzeichnet, dass** die Innengewinde (27) sich in den Umfang des Endabschnitts des Applikatorkopfes einschneidet oder einpresst.

2. Applikationsstift nach Anspruch 1, wobei der Applikatorkopf (15; 25; 45; 55; 65; 75) im eingesetzten Zustand eine prismatische oder zylindrische Grundform aufweist.

3. Applikationsstift nach einem der vorangehenden Ansprüche, wobei der Stiftkörper (11 - 81) zur Aufnahme einer harten Ampulle (13; 43) ausgebildet und seine Wandung hinreichend flexibel ist, um die im Stiftkörper aufgenommene Ampulle zu brechen und hierdurch einen Austritt der Flüssigkeit aus der Ampulle in das Innere des Stiftkörpers zu bewirken.

4. Applikationsstift nach Anspruch 3, wobei die Außenoberfläche der Wandung des Stiftkörpers (61 - 81) mit mindestens einer, insbesondere ringförmigen, Vertiefung und/oder Erhebung (61a; 61d; 61e; 61g; 71d; 71g; 81g) strukturiert ist.

5. Applikationsstift nach Anspruch 4, wobei die Außenoberfläche der Wandung des Stiftkörpers (61') eine Mehrzahl von ringförmigen Vertiefungen (61g) aufweist, welche einen Einschnürungsbereich (61e') des Stiftkörpers markieren.

6. Applikationsstift nach Anspruch 4 oder 5, wobei in die Außenoberfläche der Wandung des Stiftkörpers (61'; 81) Symbole (61f; 81f) eingeformt sind, welche eine Benutzerführung zur Ausübung von lokalem Druck auf den Stiftkörper zum Brechen der Ampulle bewirken.

7. Applikationsstift nach einem der Ansprüche 4 - 6, wobei nahe dem offenen Ende des Stiftkörpers (61; 71) eine Mehrzahl von ringförmigen Vertiefungen (61g; 71g) oder ähnlichen Strukturelementen vorgesehen ist, welche einen Greifbereich des Applikationsstiftes bestimmen.

## Claims

1. Applicator pen (10 - 80) for dispensing a liquid, particularly in a planar manner, comprising a hollow pen body (11 - 81) made of plastic with a closed end and an open end and with an applicator head (15; 25; 45; 55; 65; 75) sitting at the open end of the pen body, which applicator head (15; 25; 45; 55; 65; 75) has a porous material, particularly a foam, felt or fibre body, wherein the applicator head has an end portion fitting into the open end of the pen body, and mechanical fastening means (17; 27; 47a; 47b; 57a; 57b) for form-fit and/or frictional engagement with the outer circumference of the end portion of the applicator head are provided on the pen body and are configured in such a way that they bring about the form-fit and/or frictional engagement without substantial deformation of the end portion of the applicator head protruding into the open end of the pen body, wherein at least the end portion of the applicator head (25) and at least the open end of the pen body (21) are cylindrical in shape, and the fastening means have an inner thread (27) on the pen body, **characterized in that** the inner thread (27) cuts or presses into the circumference of the end portion of the applicator head.

2. Applicator pen according to Claim 1, wherein the applicator head (15; 25; 45; 55; 65; 75) has a prismatic or cylindrical basic shape in the inserted state.

3. Applicator pen according to either of the preceding claims, wherein the pen body (11 - 81) is configured to receive a hard ampoule (13; 43), and its wall is sufficiently flexible in order to break the ampoule received in the pen body and thereby cause the liquid to flow out of the ampoule into the interior of the pen body.

4. Applicator pen according to Claim 3, wherein the outer surface of the wall of the pen body (61 - 81) is structured with at least one in particular annular depression and/or elevation (61a; 61d; 61e; 61g; 71d; 71g; 81g).

5. Applicator pen according to Claim 4, wherein the outer surface of the wall of the pen body (61') has a plurality of annular depressions (61g) which mark a constriction region (61e') of the pen body.

6. Applicator pen according to Claim 4 or 5, wherein symbols (61f; 81f) are formed into the outer surface of the wall of the pen body (61'; 81) and guide the user to exert local pressure on the pen body in order to break the ampoule.

7. Applicator pen according to one of Claims 4 - 6, wherein a plurality of annular depressions (61g; 71g) or similar structural elements are provided near the open end of the pen body (61; 71) and define a gripping region of the applicator pen.

## Revendications

1. Crayon d'application (10 - 80) pour appliquer un liquide, en particulier à plat, avec un corps de crayon creux (11 - 81) en matière plastique présentant une extrémité fermée et une extrémité ouverte et une tête d'applicateur (15; 25; 45; 55; 65; 75) montée à l'extrémité ouverte du corps de crayon, qui présente un matériau poreux, en particulier une mousse, un feutre ou un corps fibreux, dans lequel la tête d'applicateur a une partie d'extrémité s'ajustant dans l'extrémité ouverte du corps de crayon et des moyens de fixation mécaniques (17; 27; 47a; 47b; - 57a; 57b) sont prévus sur le corps de crayon pour l'engagement par emboîtement et/ou par friction avec la périphérie extérieure de la partie d'extrémité de la tête d'applicateur et sont réalisés de telle manière qu'ils assurent l'assemblage par emboîtement et/ou par friction sans déformation notable de la partie d'extrémité de la tête d'applicateur pénétrant dans l'extrémité ouverte du corps de crayon, dans lequel au moins la partie d'extrémité de la tête d'applicateur (25) et au moins l'extrémité ouverte du corps de crayon (21) sont de forme cylindrique et les moyens de fixation présentent un filet intérieur (27) sur le corps de crayon, **caractérisé en ce que** le filet intérieur (27) entaille ou s'enfonce dans la périphérie de la partie d'extrémité de la tête d'applicateur.

2. Crayon d'application selon la revendication 1, dans lequel la tête d'applicateur (15; 25; 45; 55; 65; 75) présente dans l'état engagé une forme de base prismatique ou cylindrique.

3. Crayon d'application selon l'une quelconque des revendications précédentes, dans lequel le corps de crayon (11 - 81) est réalisé de façon à contenir une ampoule dure (13; 43) et sa paroi est suffisamment flexible, pour briser l'ampoule contenue dans le corps de crayon et provoquer ainsi un écoulement du liquide hors de l'ampoule à l'intérieur du corps de crayon.

4. Crayon d'application selon la revendication 3, dans lequel la surface extérieure de la paroi du corps de crayon (61 - 81) est structurée avec au moins un creux et/ou une surélévation, de préférence annulaire (61a; 61d; 61e; 61g; 71d; 71g; 81g).

5. Crayon d'application selon la revendication 4, dans lequel la surface extérieure de la paroi du corps de crayon (61') présente une multiplicité de creux annulaires (61g), qui marquent une zone de striction (61e') du corps de crayon.

6. Crayon d'application selon une revendication 4 ou 5, dans lequel des symboles (61f; 81f) sont formés dans la surface extérieure de la paroi du corps de crayon (61'; 81), et opèrent un guidage de l'utilisateur pour exercer une pression locale sur le corps de crayon afin de briser l'ampoule.

7. Crayon d'application selon l'une quelconque des revendications 4 à 6, dans lequel il est prévu à proximité de l'extrémité ouverte du corps de crayon (61; 71) une multiplicité de creux annulaires (61g; 71g) ou d'éléments de structure similaires, qui définissent une zone de saisie du crayon d'application.
